(19) Europäisches Patentamt
European Patent Office
Office européen des brevets

(11) Numéro de publication : **0 147 299**
**B1**

# (12) FASCICULE DE BREVET EUROPÉEN

(45) Date de publication du fascicule du brevet : **02.03.88**

(21) Numéro de dépôt : **84402623.7**

(22) Date de dépôt : **18.12.84**

(51) Int. Cl.[4] : **C 07 C 45/47**, C 07 C 45/45, C 07 C 49/813, C 07 C 49/84, C 07 C 49/83, C 07 C149/30

## (54) Procédé de préparation de benzophénones.

(30) Priorité : **22.12.83 FR 8320537**

(43) Date de publication de la demande : **03.07.85 Bulletin 85/27**

(45) Mention de la délivrance du brevet : **02.03.88 Bulletin 88/09**

(84) Etats contractants désignés : **AT BE CH DE FR GB IT LI LU NL SE**

(56) Documents cités :
**EP-A- 0 084 742**
**EP-A- 0 085 265**
**FR-A- 1 106 265**
**US-A- 2 694 729**
**Le dossier contient des informations techniques présentées postérieurement au dépôt de la demande et ne figurant pas dans le présent fascicule.**

(73) Titulaire : **RHONE-POULENC CHIMIE**
**25, quai Paul Doumer**
**F-92408 Courbevoie Cédex (FR)**

(72) Inventeur : **Desbois, Michel**
**526, Chemin du Bois**
**F-69140 - Rillieux (FR)**

(74) Mandataire : **Fabre, Madeleine-France et al**
**RHONE-POULENC INTERSERVICES Service Brevets Chimie 25, quai Paul Doumer**
**F-92408 Courbevoie Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 147 299 B1

Jouve, 18, rue St-Denis, 75001 Paris, France

**Description**

La présente invention a pour objet un procédé de préparation de benzophénones.

On connaît dans l'art antérieur des documents décrivant la préparation de ces composés.

On peut citer notamment « Olah, Friedel-Crafts and related reactions » — III — Part I — 1964 Interscience Publishers, page 8 et suivantes qui décrit la préparation de difluoro-4,4' benzophénone à partir de chlorure de fluoro-4 benzoyle et de fluorobenzène en présence de trichlorure d'aluminium. Les inconvénients de ce type de procédé résident notamment dans le fait que le chlorure de fluoro-4 benzoyle est un composé dont l'obtention est difficile. En effet, cinq étapes sont nécessaires à partir du toluène pour l'obtenir et son prix de revient en est évidemment fortement affecté. Un autre inconvénient est constitué par la grande quantité de catalyseur qu'il est nécessaire de mettre en œuvre (environ 1 mole de catalyseur pour 1 mole de substrat). De plus, ce catalyseur n'est pas recyclable et il est nécessaire de prévoir des traitements très onéreux pour le détruire afin d'éviter toute pollution.

On peut encore citer le Houben-Weyl — Tome III, 2a, page 234 qui décrit la préparation de difluoro-4,4' benzophénone à partir de fluorobenzène et de phosgène, en présence de chlorure d'aluminium comme catalyseur. On retrouve dans ce cas les mêmes inconvénients que ceux énumérés ci-dessus dérivant de la présence de chlorure d'aluminium comme catalyseur.

Il a maintenant été découvert un procédé de préparation permettant de s'affranchir de la présence de catalyseur tel que $AlCl_3$.

La présente invention concerne donc un procédé de préparation de benzophénones, caractérisé en ce que l'on fait réagir un dérivé benzénique désactivé avec un composé de formule générale :

$$COX_1X_2 \qquad (I)$$

où $X_1$ et $X_2$ identiques ou différents représentent un atome d'halogène, dans l'acide fluorhydrique liquide et en présence de trifluorure de bore en quantité telle que la pression absolue de trifluorure de bore dans l'enceinte réactionnelle soit d'au moins 1 bar.

Au sens de la présente invention, on entend par dérivé benzénique désactivé le composé de formule générale (II) :

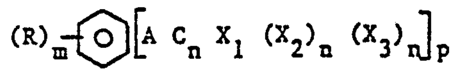

(II)

dans lequel

A représente une liaison covalente, un atome d'oxygène ou de soufre

$X_1$, $X_2$ et $X_3$ représentent chacun un atome d'halogène identique ou différent

n est compris entre 0 et 2 et lorsque A est une liaison covalente, n est égal à 0

p est égal à 1 ou 2

R est choisi parmi le groupe comprenant l'hydrogène et les radicaux halogéno, hydroxyles, alkyle, phényle, alkoxy, alkylthio.

m est égal à 1 ou 2.

Selon un mode de réalisation préféré de l'invention dans la formule II, R est l'hydrogène et le dérivé benzénique est alors représenté par un halogénobenzène, un perhalogénoalkoxy benzène ou un perhalogénoalkylthiobenzène.

Encore plus préférentiellement, le dérivé benzénique est choisi parmi les halogénobenzènes et tout particulièrement le fluorobenzène.

L'invention est particulièrement bien adaptée à la mise en œuvre de $COCl_2$ car il est parmi les composés de formule I celui qui est le plus facilement accessible industriellement.

L'acide fluorhydrique utilisé est de préférence anhydre.

La mise en œuvre d'acide fluorhydrique aqueux entraînerait une consommation inutile de trifluorure de bore sous forme de HF, $BF_3$, $H_2O$ ($H_3O^+BF_4^-$).

Il est préférable de travailler avec une quantité de $BF_3$ telle que la pression absolue de $BF_3$ dans l'enceinte réactionnelle soit comprise entre 6 et 20 bars.

Plus la pression sera élevée, plus la vitesse de réaction augmentera. Une pression supérieure à 20 bars n'est pas exclue du domaine de l'invention mais n'apporte pas d'avantage particulier. L'homme de l'art adoptera donc la pression à l'économie du procédé.

Avantageusement, le rapport molaire de l'acide fluorhydrique au dérivé benzénique est compris entre 5 et 50. Encore plus préférentiellement ce rapport est compris entre 10 et 30.

Le rapport molaire du dérivé benzénique au composé de formule I est, de préférence, compris entre 1 et 3. Encore préférentiellement, ce rapport est égal à environ 2.

La température est comprise, de préférence, entre 20 et 150 °C.

Les benzophénones obtenues répondent à la formule générale suivante :

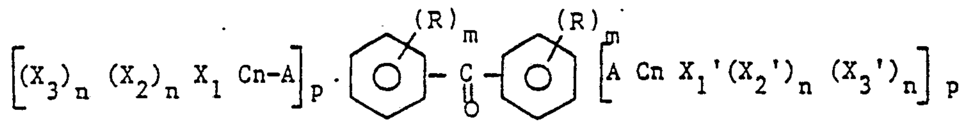

dans laquelle A, R, n, m, p ont les significations précédentes

si n = 0 X a la signification précédente

si n = 1 X = F car il est échangé avec les ions fluor du milieu réactionnel.

si n = 2 les benzophénones obtenues répondent à la formule suivante

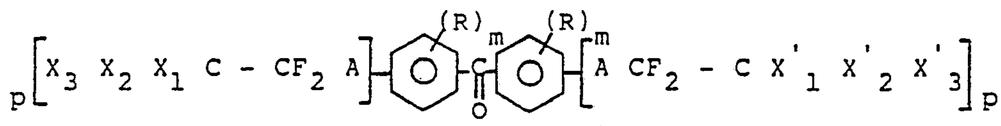

dans laquelle X a la signification précédente, l'échange entre les halogènes et les ions fluor du milieu réactionnel n'ayant lieu que sur les halogènes fixés sur le carbone situé en α de l'hétéroatome.

Parmi les dérivés benzéniques de départ, on peut citer : le fluorobenzène, le chlorobenzène, le bromobenzène, l'iodobenzène, les difluorobenzènes, les dichlorobenzènes, les dibromobenzènes, les chlorofluorobenzènes, les chlorobromobenzènes, les bromofluorobenzènes, le trifluorométhoxybenzène, le trichlorométhoxybenzène, le tribromométhoxybenzène, le bromodichlorométhoxybenzène, le bromodifluorométhoxybenzène, les chlorotrifluorométhoxybenzènes, les fluorotrifluorométhoxybenzènes, le trifluorométhylthiobenzène, le trichlorométhylthiobenzène, les chlorotrifluorométhylthiobenzènes, les fluorotrifluorométhylthiobenzènes, le pentafluoroéthoxybenzène, le pentachloroéthoxybenzène, les fluorotoluènes, les chlorotoluènes, les fluoroanisoles, les chloroanisoles, les fluorothioanisoles, les chlorophénols, les fluorophénols, les trifluorométhoxyphénols, les trifluorométhylthiophénols.

Parmi les benzophénones obtenues par le procédé de l'invention on peut citer :

La difluoro-4,4' benzophénone, la dichloro-4,4' benzophénone, la dibromo-4,4' benzophénone, la bis-trifluorométhoxy-4,4' benzophénone, la bis-trifluorométhylthio-4,4' benzophénone, la bis-(difluoro-α,α trichloro-β,β,β éthoxy)-4,4' benzophénone, les difluorodiméthylbenzophénones, les difluoro-dihydroxy benzophénones, les difluoro diméthoxy benzophénones, les tétrafluorobenzophénones, les tétrachlorobenzophénones.

Les benzophénones obtenues par le procédé précédemment décrit sont utilisées comme intermédiaires de synthèse dans l'industrie pharmaceutique, phytosanitaire et des polymères (brevet allemand N° 2 659 580 et US N° 3 732 307).

L'invention va maintenant être plus complètement décrite à l'aide des exemples qui vont suivre.

## Exemple 1

Dans un réacteur en acier inoxydable agité magnétiquement et refroidi aux environs de 0 °C par un bain d'eau glacée, on introduit 100 g (5 moles) d'acide fluorhydrique anhydre, 9,6 g (0,1 mole) de fluorobenzène et 5 g (0,05 mole) de phosgène.

Le réacteur est fermé puis pressurisé avec 6 bars de trifluorure de bore. Le mélange réactionnel est alors porté sous agitation à 80 °C pendant 3 heures. Après refroidissement à température ambiante et décompression, le brut réactionnel est coulé sur 200 g de glace, le mélange hétérogène ainsi obtenu est extrait par 3 fois 100 $cm^3$ de chlorure de méthylène. La phase organique est lavée 2 fois par 150 $cm^3$ d'eau puis séchée. Après évaporation, on recueille 7,2 g (rendement : 66 %) d'un composé contenant 75 % de difluoro-4,4' benzophénone et 25 % de difluoro-2,4' benzophénone (analyses chromatographie phase liquide).

## Exemple 2

On procède d'une manière analogue à l'exemple 1 avec les composés suivants et dans les mêmes conditions :

| | |
|---|---|
| acide fluorhydrique | 100 g (5 m) |
| trifluorométhoxybenzène | 32,4 g (0,2 m) |
| phosgène | 9,9 g (0,1 m) |
| $BF_3$ | 6 bars à 20 °C |
| Température | 80 °C |
| Durée | 3 H 40 mn |

On recueille 14,4 g (rendement : 41,1 %) d'un mélange essentiellement composé de bis-trifluorométhoxy-4,4' et de bis-trifluorométhoxy-2,4' benzophénone (Analyses chromatographie phase gazeuse, infrarouge et spectrométrie de masse).

Exemple 3

On procède d'une manière analogue à l'exemple 1 avec les composés et conditions suivants :

| | |
|---|---|
| acide fluorhydrique | 50 g (2,5 m) |
| chlorobenzène | 11,2 g (0,1 m) |
| phosgène | 6,5 g (0,065 m) |
| $BF_3$ | 10 bars (à 20 °C) |
| température | 120 °C |
| durée | 23 H 15 mn |

On recueille 7 g (rendement ≃ 55 %) d'un mélange essentiellement composé de dichloro-4,4' et dichloro-2,4' benzophénones (Analyses CPG et IR).

Exemple 4

On procède d'une manière analogue à l'exemple 1 avec les composés et conditions suivants :

| | |
|---|---|
| acide fluorhydrique | 50 g (2,5 m) |
| m-dichlorobenzène | 14,7 g (0,1 m) |
| phosgène | 6,5 g (0,065 m) |
| $BF_3$ | 10 bars à 20 °C |
| température | 120 °C |
| durée | 23 H 00 |

Par analyse IR et CGP sur le composé brut, on note la présence d'environ 7 % d'un mélange de tétrachlorobenzophénones.

**Revendications**

1. Procédé de préparation de benzophénones, dans l'acide fluorhydrique liquide en présence de trifluorure de bore caractérisé en ce que l'on fait réagir un composé de formule $COX_1X_2$ (I) où $X_1$, et $X_2$, identiques ou différents représentent un atome d'halogène avec un dérivé benzénique désactivé.

2. Procédé selon la revendication 1 caractérisé en ce que le dérivé benzénique a pour formule générale :

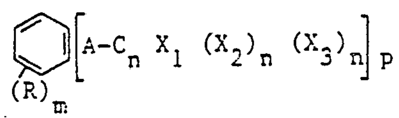 (II)

dans laquelle

A représente une liaison covalente, un atome d'oxygène ou de soufre

$X_1$, $X_2$, $X_3$ représentent chacun un atome d'halogène identique ou différent

n est compris entre 0 et 2 et lorsque A est une liaison covalente n est égal à 0

R est choisi parmi le groupe comprenant l'hydrogène et les radicaux halogèno, hydroxyle, alkyle, alkoxy, phényle, alkylthio.

m est égal à 1 ou 2

p est égal à 1 ou 2

3. Procédé selon la revendication 2 caractérisé en ce que dans la formule (II) R est l'hydrogène.

4. Procédé selon les revendications 2 et 3 caractérisé en ce que dans la formule (II) n est égal à 0.

5. Procédé selon la revendication 4 caractérisé en ce que le dérivé benzénique est le fluorobenzène.

6. Procédé selon la revendication 1, caractérisé en ce que dans la formule (I), $X_1$ et $X_2$ représente le Cl.

7. Procédé selon l'une quelconque des revendications précédentes, caractérisé en ce que l'acide fluorhydrique est anhydre.

8. Procédé selon l'une quelconque des revendications précédentes, caractérisé en ce que l'on utilise une quantité de trifluorure de bore telle que la pression absolue de $BF_3$ dans l'enceinte réactionnelle soit comprise entre 6 et 20 bars.

9. Procédé selon l'une quelconque des revendications précédentes, caractérisé en ce que l'on utilise une quantité d'acide fluorhydrique tel que le rapport molaire de l'acide fluorhydrique au dérivé benzénique soit compris entre 5 et 50.

10. Procédé selon l'une quelconque des revendications précédentes, caractérisé en ce que le

rapport molaire du dérivé benzénique au composé de formule I soit compris entre 1 et 3.

11. Procédé selon l'une quelconque des revendications précédentes, caractérisé en ce que l'on opère à une température comprise entre 20 et 150 °C.

**Claims**

1. Process for preparing benzophenones in liquid hydrofluoric acid in the presence of boron trifluoride, characterized in that a compound of formula $COX_1X_2$ (I), where $X_1$ and $X_2$, which may be identical or different, denote a halogen atom, is reacted with a deactivated benzene derivative.

2. Process according to Claim 1, characterized in that the benzene derivative has the general formula :

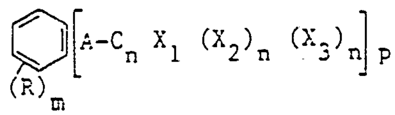

(II)

in which

A denotes a covalent bond or an oxygen or sulphur atom

$X_1$, $X_2$, $X_3$ each denote an identical or different halogen atom

n is between 0 and 2, and when A is a covalent bond n equals 0

R is chosen from the group comprising hydrogen and halo, hydroxyl, alkyl, alkoxy, phenyl and alkylthio radicals

m equals 1 or 2

p equals 1 or 2.

3. Process according to Claim 2, characterized in that, in the formula (II), R is hydrogen.

4. Process according to Claims 2 and 3, characterized in that, in the formula (II) n equals 0.

5. Process according to Claim 4, characterized in that the benzene derivative is fluorobenzene.

6. Process according to Claim 1, characterized in that, in the formula (I), $X_1$ and $X_2$ denote Cl.

7. Process according to any one of the preceding claims, characterized in that the hydrofluoric acid is anhydrous.

8. Process according to any one of the preceding claims, characterized in that an amount of boron trifluoride is used such that the absolute pressure of $BF_3$ in the reaction chamber is between 6 and 20 bars.

9. Process according to any one of the preceding claims, characterized in that an amount of hydrofluoric acid is used such that the mole ratio of hydrofluoric acid to the benzene derivative is between 5 and 50.

10. Process according to any one of the preceding claims, characterized in that the mole ratio of the benzene derivative to the compound of formula I is between 1 and 3.

11. Process according to any one of the preceding claims, characterized in that the reaction is performed at a temperature of between 20 and 150 °C.

**Patentansprüche**

1. Verfahren zur Herstellung von Benzophenonen in flüssiger Fluorwasserstoffsäure in Gegenwart von Bortrifluorid, dadurch gekennzeichnet, daß man eine Verbindung der Formel $COX_1X_2$ (I), bei der $X_1$ und $X_2$ identisch oder verschieden sind und ein Halogenatom darstellen, mit einem desaktivierten Benzolderivat reagieren läßt.

2. Verfahren nach Anspruch 1, dadurch gekennzeichnet, daß das Benzolderivat die allgemeine Formel hat :

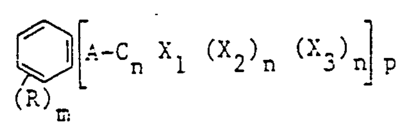

(II)

in der

A eine covalente Bindung, ein Sauerstoffatom oder Schwefelatom darstellt

$X_1$, $X_2$, $X_3$ jeweils ein Halogenatom darstellen, identisch oder verschieden,

n = 0, 1 oder 2 ist und wenn A eine covalente Bindung ist n gleich 0 ist,

R ausgewählt wird aus der Gruppe umfassend Wasserstoff und die Reste Halogen, Hydroxyl, Alkyl, Alkoxy, Phenyl, Alkylthio,

m gleich 1 oder 2 ist,

p gleich 1 oder 2 ist.

3. Verfahren nach Anspruch 2, dadurch gekennzeichnet, daß in der Formel (II) R ein Wasserstoffatom ist.

4. Verfahren nach den Ansprüchen 2 und 3, dadurch gekennzeichnet, daß in der Formel (II) n gleich 0 ist.

5. Verfahren nach Anspruch 4, dadurch gekennzeichnet, daß das Benzolderivat Fluorbenzol ist.

6. Verfahren nach Anspruch 1, dadurch gekennzeichnet, daß in der Formel (I) $X_1$ und $X_2$ Cl darstellen.

7. Verfahren nach einem der vorhergehenden Ansprüche, dadurch gekennzeichnet, daß die Fluorwasserstoffsäure wasserfrei ist.

8. Verfahren nach einem der vorhergehenden Ansprüche, dadurch gekennzeichnet, daß man eine solche Menge Bortrifluorid verwendet, daß der absolute Druck von $BF_3$ im Reaktionsbehälter zwischen 6 und 20 bar beträgt.

9. Verfahren nach einem der vorhergehenden Ansprüche, dadurch gekennzeichnet, daß man eine solche Menge Fluorwasserstoffsäure verwendet, daß das molare Verhältnis von Fluorwasserstoffsäure zu Benzolderivat zwischen 5 und 50 liegt.

10. Verfahren nach einem der vorhergehenden Ansprüche, dadurch gekennzeichnet, daß das molare Verhältnis von dem Benzolderivat zu der Verbindung der Formel I zwischen 1 und 3 liegt.

11. Verfahren nach einem der vorhergehenden Ansprüche, dadurch gekennzeichnet, daß man bei einer Temperatur zwischen 20 °C und 150 °C arbeitet.